# EUROPEAN PATENT APPLICATION

(11) **EP 3 733 135 A1**
(43) Date of publication of application: **04.11.2020**
(21) Application number: 18893373.3
(22) Date of filing: 21.12.2018
(51) Int. Cl.: A61F 2/89, A61F 2/07, A61F 2/95

(54) **TUBULAR IMPLANTED APPLIANCE AND DEVICE FOR IMPLANTING TUBULAR IMPLANTED APPLIANCE**

(30) Priority: 28.12.2017 JP 2017253190
(71) Applicant: Kawasumi Laboratories, Inc., Saiki-shi, Oita 876-0121 (JP)
(72) Inventor: HIDARI, Kentaro, Ono-shi, Oita 879-7153 (JP); YUBA, Toshiyasu, Ono-shi, Oita 879-7153 (JP); YOKOTA, Tomoaki, Ono-shi, Oita 879-7153 (JP)
(74) Representative: LKGLOBAL Lorenz & Kopf PartG mbB Patentanwälte
(86) International application number: PCT/JP2018/047390
(87) International publication number: WO 2019/131559

(57) **Abstract**

A tubular implanted appliance (103) is provided with a fr amework part (2) and a tubular coating film part (1) forming a partitio n wall, the tubular implanted appliance (103) being configured so as to be able to expand and retract in the axial direction of the coating film part. The framework part has a plurality of annular linear members (2 1) extending along the circumferential direction of the coating film part while bending in the axial direction of the coating film part. The plur ality of linear members are disposed in a row in the axial direction of the coating film part, and are attached and fixed to the surface of the coating film part at intermediate portions (21c) between end parts in t he axial direction of the linear members.

## Description

### TECHNICAL FIELD

The present invention relates to a tubular implanted appliance and a device for implanting a tubular implanted appliance.

### BACKGROUND ART

Conventionally, tubular implanted appliances are used for the treatment of aortic aneurysms or aortic dissections that occur in the aorta (for example, see Patent Document 1). In this method, for example, an implanting device that holds a tubular implanted appliance in the contracted states inside a sheath is used to release the tubular implanted appliance from the end of the sheath inside the aorta, which implants the tubular implanted appliance at the treatment site and prevents the rupture of the aortic aneurysm.

### PRIOR ART DOCUMENT

### PATENT DOCUMENT

Patent Document 1: Japanese Unexamined Patent Application Publication No. 2010-268950

### SUMMARY OF THE INVENTION

### PROBLEMS TO BE SOLVED BY THE INVENTION

However, because the size of a treatment site varies from patient to patient, it is desirable to implant a tubular implanted appliance having optimal dimensions (particularly with respect to the axial direction length dimension) for the treatment site of each patient. For example, if the axial direction length of the tubular implanted appliance is too short, the coverage of the treatment site is insufficient, while if the axial direction length of the tubular implanted appliance is too long, there is a concern that the tubular implanted appliance may also cover a site where coverage is not necessary (such as the vessel opening of branch vessel).

That is to say, in order to handle treatment sites having a variety of different sizes on a per-patient basis, it is not sufficient for the axial direction length of the tubular implanted appliance to be simply made shorter or longer, and this causes the problem of requiring tubular implanted appliances having a plurality of different dimensions to be prepared in advance.

Such problems may also occur with respect to tubular implanted appliances intended to be implanted in a tubular tissue other than a blood vessel (for example, the digestive tract or the bile duct).

An object of the present invention is to provide a tubular implanted appliance which is capable of handling treatment sites having a variety of different sizes on a per-patient basis.

### MEANS FOR SOLVING THE PROBLEM

A tubular implanted appliance according to the present invention includes a framework part and a tubular coating film part forming a partition wall, the tubular implanted appliance being capable of expanding and contracting in an axial direction of the coating film part, in which the framework part has a plurality of annular linear members extending along a circumferential direction of the coating film part while bending in the axial direction of the coating film part, and the plurality of linear members are disposed in a row in the axial direction of the coating film part, and are attached and fixed to a surface of the coating film part at intermediate portions between both of end parts in the axial direction of the linear members.

### EFFECT OF THE INVENTION

According to the present invention, treatment sites having a variety of different sizes can be handled on a per-patient basis without preparing tubular implanted appliances having a plurality of dimensions in advance.

### BRIEF DESCRIPTION OF THE DRAWINGS

[FIG. 1] FIG. 1(a) and FIG. 1(b) are diagrams schematically showing a device for implanting a tubular implanted appliance of an embodiment according to the present invention.
[FIG. 2] FIG. 2(a) and FIG. 2(b) are perspective views showing a tubular implanted appliance provided in the device for implanting a tubular implanted appliance shown in FIG. 1(a) and FIG. 1(b).
[FIG. 3] FIG. 3 is a diagram showing enlarged the section of the tubular implanted appliance shown in FIG. 2(b) enclosed by the broken line A.
[FIG. 4] FIG. 4 is a diagram relating to a modification of the embodiment of the present invention, and corresponds to the tubular implanted appliance shown in FIG. 3.
[FIG. 5] FIG. 5(a) and FIG. 5(b) are diagrams relating to another modification of the embodiment of the present invention, and respectively correspond to the tubular implanted appliance shown in FIG. 2(a) and FIG. 2(b).

### DESCRIPTION OF THE EMBODIMENT

Hereinafter, an embodiment of the present invention will be described with reference to the drawings.

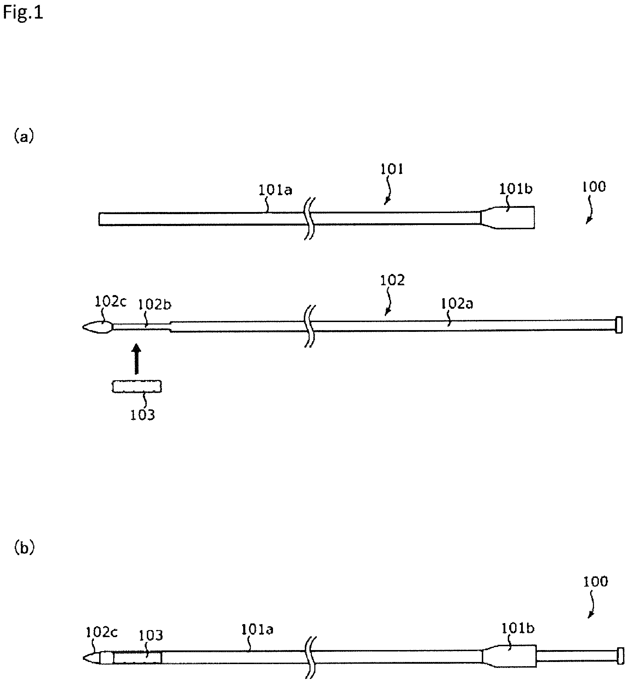

A device for implanting a tubular implanted appliance 100 will firstly be described with reference to FIG. 1(a) and FIG. 1(b).

FIG. 1(a) is a diagram showing the members that constitute the device for implanting a tubular implanted appliance 100 according to the present embodiment, and FIG. 1(b) is a diagram illustrating a state in which the members have been assembled.

In FIG. 1(a) and FIG. 1(b), the size (such as the length and diameter dimensions) and shape of the members that constitute the device for implanting a tubular implanted appliance 100 are schematically illustrated.

For example, as shown in FIG. 1(a) and FIG. 1(b), the device for implanting a tubular implanted appliance 100 is a device used to implant a tubular implanted appliance 103 inside a blood vessel of the thoracic aorta, and specifically includes a tubular sheath 101, an inner rod 102 disposed on the inside of the sheath 101 which is configured so as to be capable of moving forward and backward along the axial direction (longitudinal direction), and a tubular implanted appliance 103. In the present example, the tubular implanted appliance 103 is designed assuming that it will be implanted inside a blood vessel, and is also referred to as a stent graft.

The sheath 101 includes a tubular sheath body 101a and a hub 101b provided on the proximal end side of the sheath body 101a. Here, although not illustrated, the hub 101b is provided with a nut for securing the inner rod 102 to the sheath 101 and for releasing the secured state.

The sheath 101 is formed of a flexible material. Examples of the flexible material include biocompatible synthetic resins (elastomers) selected from fluororesins, polyamide-based resins, polyethylene-based resins, polyvinyl chloride-based resins and the like, resin compounds in which another material is mixed with these resins, multilayered structures made of these synthetic resins, and composites of these synthetic resins and metal wires.

The inner rod 102 includes a rod-shaped rod body 102a, a holder 102b that holds the tubular implanted appliance 103 in the contracted state, and a distal end tip 102c provided on the front end (distal end) of the inner rod 102. The diameter of the holder 102b is, for example, set to be narrower than that of the rod body 102a by the amount of the thickness of the tubular implanted appliance 103.

Examples of the material forming the rod body 102a and the holder 102b include various materials having appropriate hardness and flexibility, such as resins (plastics or elastomers) and metals. Examples of the material forming the distal end tip 102c include various materials having appropriate hardness and flexibility, such as synthetic resins (elastomers) selected from polyamide-based resins, a polyurethane-based resins, polyvinyl chloride-based resins and the like.

Although not illustrated, the rod body 102a, the holder 102b, and the distal end tip 102c are provided with, for example, a lumen for passing a guide wire, a lumen for passing a trigger wire for expanding the tubular implanted appliance 103 in the contracted state at the treatment site, and the like, which are formed along the axial direction of the inner rod 102.

### <Tubular Implanted Appliance>

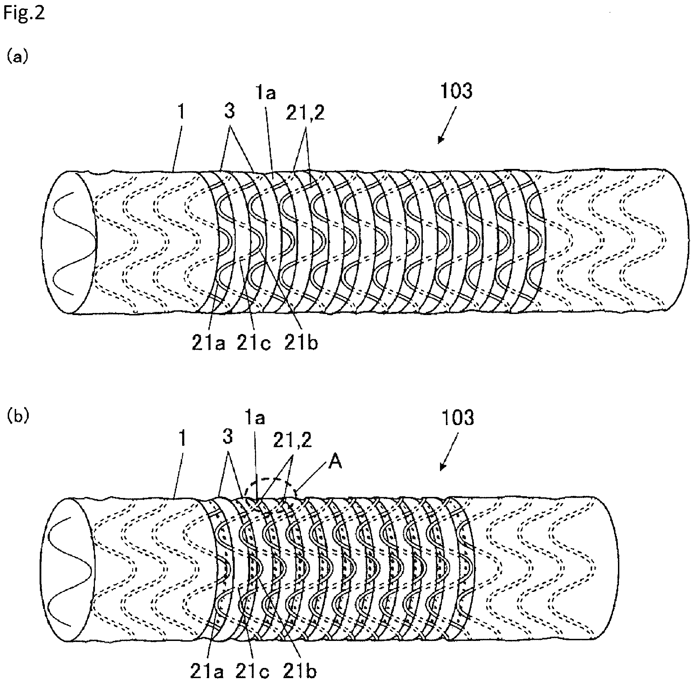

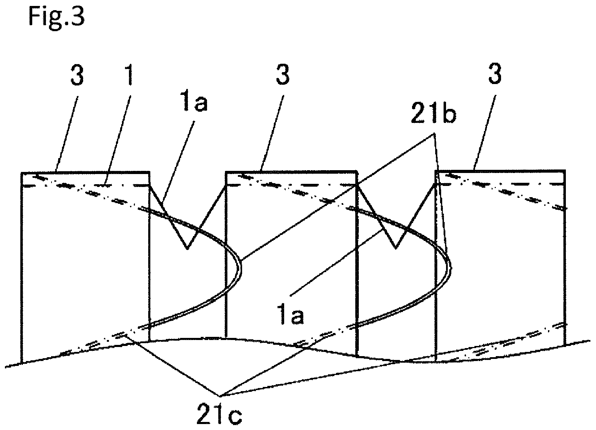

Next, the tubular implanted appliance 103 will be described with reference to FIG. 2(a), FIG. 2(b), and FIG. 3.

FIG. 2(a) is a perspective view schematically showing the tubular implanted appliance 103, and FIG. 2(b) is a perspective view schematically showing a state in which the tubular implanted appliance 103 is contracted in the axial direction. Furthermore, FIG. 3 is a diagram showing enlarged the section of the tubular implanted appliance 103 shown in FIG. 2(b) enclosed by the broken line A.

For example, as shown in FIG. 2(a), FIG. 2(b), and FIG. 3, the tubular implanted appliance 103 has a cover parts 3 attached to the outer surface of a tubular coating film part 1 so as to sandwich the framework part 2, which is composed of linear members 21.

The coating film part 1 forms a partition wall that separates a lumen serving as a blood flow path from the outer region of the tubular implanted appliance 103. Examples of the material of the coating film part 1 include fluorine resins such as PTFE (polytetrafluoroethylene) and polyester resins such as polyethylene terephthalate.

Furthermore, the framework part 2 having the plurality of linear members 21, ... is disposed on the outer surface of the coating film part 1.

The framework part 2 is configured so as to be deformable from the contracted state, in which it is contracted inside in the radial direction, to an expanded state, in which it is expanded outside in the radial direction. Moreover, the framework part 2 has a plurality of linear members 21, ..., which are arranged at predetermined intervals (for example, at equal intervals) in the axial direction of the coating film part 1 (the horizontal direction in FIG. 2(a))

The plurality of linear members 21, ... have an annular shape that extends along the circumferential direction of the coating film part 1, while being bent so as to have bent parts 21a and 21b in the axial direction of the coating film part 1. In addition, the plurality of linear members 21, ... are disposed such that, between adjacent members in the axial direction of the coating film part 1, the bent parts 21a of one side do not face the bent parts 21b of the other side, that is to say, the positions of the peaks and the positions of the valleys of adjacent linear members 21 are made to coincide in the circumferential direction.

Furthermore, each linear member 21 has bent parts 21a on one side in the axial direction of the coating film part 1 (for example, the left side in FIG. 2(a)), bent parts 21b on another side in the axial direction (for example, the right side in FIG. 2(a)), and intermediate portions 21c that connect the bent parts 21a and 21b. Specifically, the intermediate portions 21c are continuously provided on the another side ends (for example, the right side in FIG. 2(a)) of the bent parts 21a, which are on the one side (for example, the left side in FIG. 2(a)), and the bent parts 21b, which are on the another side, are continuously provided on the another side ends of the intermediate portions 21c.

Although not illustrated, the both of end parts of each linear member 21 are, for example, connected by a connecting means such as caulking to form an annular shape as a whole.

Moreover, each linear member 21 is attached and fixed to the outer surface of the coating film part 1 between both of end parts in the axial direction of the coating film part 1, that is to say, at the intermediate portions 21c between the bent parts 21a and 21b.

Specifically, a plurality of cover parts 3, ... that cover the intermediate portions 21c of the linear members 21, ... are attached to the coating film part 1. The cover parts 3 are made of, for example, a ribbon-shaped tape extending in the circumferential direction of the coating film part 1, and the intermediate portions 21c of each of the plurality of linear members 21, ... are attached and fixed to the surface of the coating film part 1 as a result of the cover parts 3 being attached and fixed to the surface of the coating film part 1 so as to cover the intermediate portions 21c.

Consequently, the rigidity is relatively low at the exposed portions 1a of the coating film part 1 where the plurality of linear members 21 are not attached and fixed, or more specifically, at the exposed portions 1a where the cover parts 3 are not attached so as to cover the plurality of linear members 21, .... For this reason, for example, when an external force acts on the tubular implanted appliance 103 in the axial direction, the exposed portions 1a of the coating film part 1 contracts in the axial direction, and the entire tubular implanted appliance 103 contracts in the axial direction.

Furthermore, because the bent parts 21a and 21b of the linear members 21 are located on the outer radial direction side of the exposed portions 1a of the coating film part 1, deformations toward the outer radial direction of the exposed portions 1a are restricted when the exposed portions 1a contract in the axial direction. That is to say, when the entire tubular implanted appliance 103 is contracted in the axial direction, the exposed portions 1a of the coating film part 1 become deformed and folded inside in the radial direction (see FIG. 3).

Here, for example, although not illustrated, a crease or the like that induces folding may be formed on the exposed portions 1a of the coating film part 1 so that the exposed portions 1a can more easily fold inside in the radial direction.

Furthermore, when the tubular implanted appliance 103 is not in the contracted state (see FIG. 2(a)), the axial direction length of the bent parts 21a and 21b of one linear member 21, which are not covered by the cover parts 3, is regulated such that the length is equal to the spacing between the cover part 3 corresponding to the one linear member 21 and another cover part 3 which is adjacent in the axial direction, or is shorter than this spacing. Further, when the tubular implanted appliance 103 is in a contracted state (see FIG. 2(b)), the axial direction length of the bent parts 21a and 21b is regulated such that the length is longer than the spacing between the cover part 3 corresponding to the one linear member 21 and another cover part 3 which is adjacent in the axial direction.

As a result, when the tubular implanted appliance 103 is in a state where it is contracted in the axial direction of the coating film part 1, among the plurality of linear members 21, ..., the axial direction ends of the bent parts 21a and 21b of the one linear member 21 overlap with the cover part 3 corresponding to the another linear member 21, which is adjacent to the one linear member 21, and become disposed on the surface thereof.

As described above, because the tubular implanted appliance 103 is configured to be capable of expanding and contracting in the axial direction of the coating film part 1, when the tubular implanted appliance 103 is implanted inside a blood vessel of a patient, it is preferable for discharge from the device for implanting a tubular implanted appliance 100 to be performed in a state where the axial direction length of the tubular implanted appliance 103 is regulated so as to correspond to the size of the treatment site.

That is to say, because the tubular implanted appliance 103 is held inside the sheath 101 in a state where it is contracted in the radial direction, the expansion force of the tubular implanted appliance 103 becomes a discharge resistance when discharge is performed by moving the sheath 101 with respect to the tubular implanted appliance 103. As a result, the tubular implanted appliance 103 becomes elongated in the axial direction, and the axial direction length of the tubular implanted appliance 103 can no longer be made to correspond to the size of the treatment site of the patient. For this reason, although not illustrated, the device for implanting a tubular implanted appliance 100 is preferably provided with, for example, a regulating device that regulates the axial direction length of the tubular implanted appliance 103 until the tubular implanted appliance 103 is completely discharged.

As described above, the device for implanting a tubular implanted appliance 100 of the present embodiment is provided with a tubular implanted appliance 103 including a framework part 2, and a tubular coating film part 1 forming a partition wall, the tubular implanted appliance 103 being capable of expanding and contracting in an axial direction of the coating film part 1. The framework part 2 has a plurality of annular linear members 21, ... extending along a circumferential direction of the coating film part 1 while bending in the axial direction of the coating film part 1, and the plurality of linear members 21, ... are disposed in a row in the axial direction of the coating film part 1, and are attached and fixed to a surface of the coating film part 1 at intermediate portions 21c between both of end parts in the axial direction of the linear members 21.

Therefore, because both of end parts of the linear members 21 in the axial direction of the coating film part 1 (for example, the bent parts 21a and 21b), which are the sections other than the intermediate portions 21c, are not attached and fixed to the coating film part 1, the axial direction length of the tubular implanted appliance 103 can be adjusted by contracting, in the axial direction, the sections of the coating film part 1 to which the plurality of linear members 21, ... are not attached and fixed (exposed portions 1a). Specifically, as a result of the sections of the coating film part 1 to which the plurality of linear members 21, ... are not attached and fixed being folded inside in the radial direction, the tubular implanted appliance 103 can be contracted in the axial direction.

As a result, treatment sites of a variety of different sizes can be handled on a per-patient basis without preparing tubular implanted appliances 103 having a plurality of dimensions in advance.

Furthermore, when the tubular implanted appliance 103 is in a state where it is contracted in the axial direction, among the plurality of linear members 21, ..., because the axial direction ends of the one linear member 21 overlap with the cover part 3 of the another linear member 21, which is adjacent to the one linear member 21, and become disposed on the surface thereof, the sections of each of the plurality of linear members 21, ... which are not covered by the plurality of cover parts 3 do not become an obstruction in a state where there tubular implanted appliance 103 is contracted in the axial direction, and therefore, the tubular implanted appliance 103 can be appropriately contracted in the axial direction.

The present invention is not limited to the embodiment described above, and various improvements and design changes may be made without departing from the spirit of the present invention.

For example, although an example of a tubular implanted appliance 103 in which the linear members 21 of the framework part 2 are attached and fixed to the outer surface of the coating film part 1 has been illustrated, examples are not limited to this, and the linear members 21 may have a configuration in which they are attached and fixed to the inner surface of the coating film part 1, or a configuration in which they are woven into the coating film part 1.

Moreover, although the embodiment described above illustrated an example where the linear members 21 are attached and fixed to the coating film part 1 by attaching the cover parts 3 so as to cover the intermediate portions 21c of the linear members 21, examples are not limited to this and may be arbitrarily modified as appropriate. That is to say, for example, it is not always necessary to use the cover parts 3, and the intermediate portions 21c of the linear members 21 may be fixed by being sewn to the coating film part 1.

Furthermore, although the embodiment described above illustrated an example of a configuration in which the framework part 2 has a plurality of linear members 21, ..., examples are not limited to this and may be arbitrarily modified as appropriate.

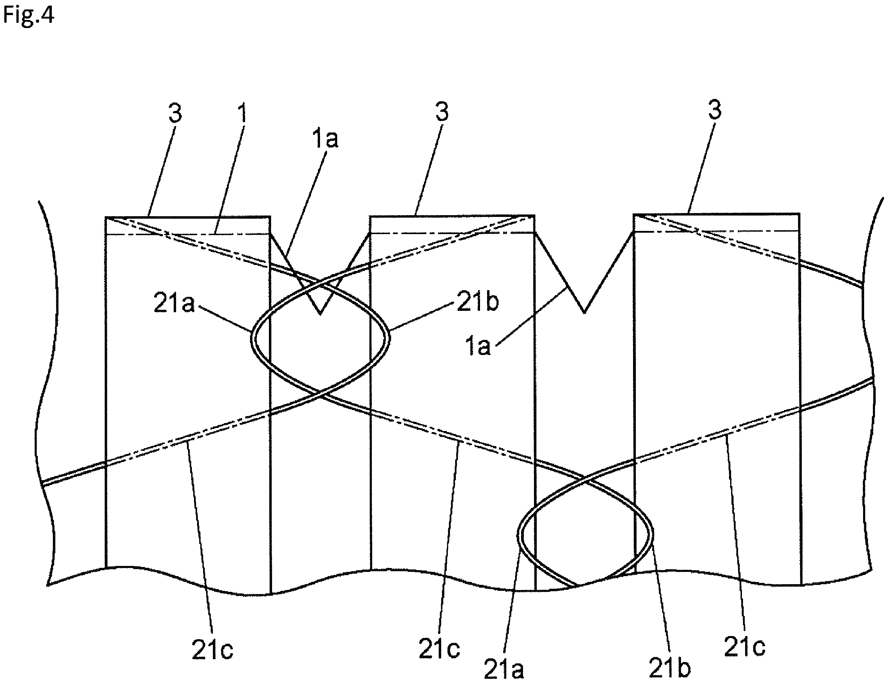

For example, in the embodiment described above, the plurality of linear members 21, ... are disposed such that, between adjacent members in the axial direction of the coating film part 1, the bent parts 21a of one side do not face the bent parts 21b of the other side (see FIG. 3). However, examples of the arrangement structure of the plurality of linear members 21, ... are not limited to this, and as shown in FIG. 4, for example, the plurality of linear members 21, ... may be woven (combined) such that, between adjacent members in the axial direction of the coating film part 1, the bent parts 21a of one side engage the bent parts 21b of the other side.

That is to say, the tubular implanted appliance 103 may have a configuration including a framework part 2 and a tubular coating film part 1 forming a partition wall, the tubular implanted appliance 103 being capable of expanding and contracting in an axial direction of the coating film part 1, wherein the framework part 2 is formed by weaving linear members 21 in a manner that bent parts 21a and 21b thereof, which bend in the axial direction of the coating film part 1, mutually engage, and is attached and fixed to a surface of the coating film part 1 at sections other than the bent parts 21a and 21b of the linear members 21.

Even with such a configuration, in a similar manner to the embodiment described above, the axial direction length of the tubular implanted appliance 103 can be adjusted, and treatment sites of a variety of different sizes can be handled on a per-patient basis without preparing tubular implanted appliances 103 having a plurality of dimensions in advance.

Similarly, the tubular implanted appliance 103 may have a configuration including a framework part 2 and a tubular coating film part 1 forming a partition wall, the tubular implanted appliance 103 being capable of expanding and contracting in an axial direction of the coating film part 1, wherein the framework part 2 has a linear member (not illustrated) that spirally extends along a circumferential direction of the coating film part 1 while bending in an axial direction of the coating film part 1, and the linear member, which has single units equivalent to one circumference of the coating film part 1, is attached and fixed to a surface of the coating film part 1 at each of the single units at intermediate portions (not illustrated) between both of the end parts in the axial direction.

Further, in the above embodiment, although a tubular implanted appliance 103 for the thoracic aorta was illustrated, the present invention is not limited to this, and although not illustrated, it is also applicable, for example, to a tubular implanted appliance for the abdominal aorta or a tubular implanted appliance for the thoracic abdominal aorta. Moreover, the present invention is also applicable to tubular implanted appliances intended to be implanted in organs other than a blood vessel (such as the digestive tract or the bile duct).

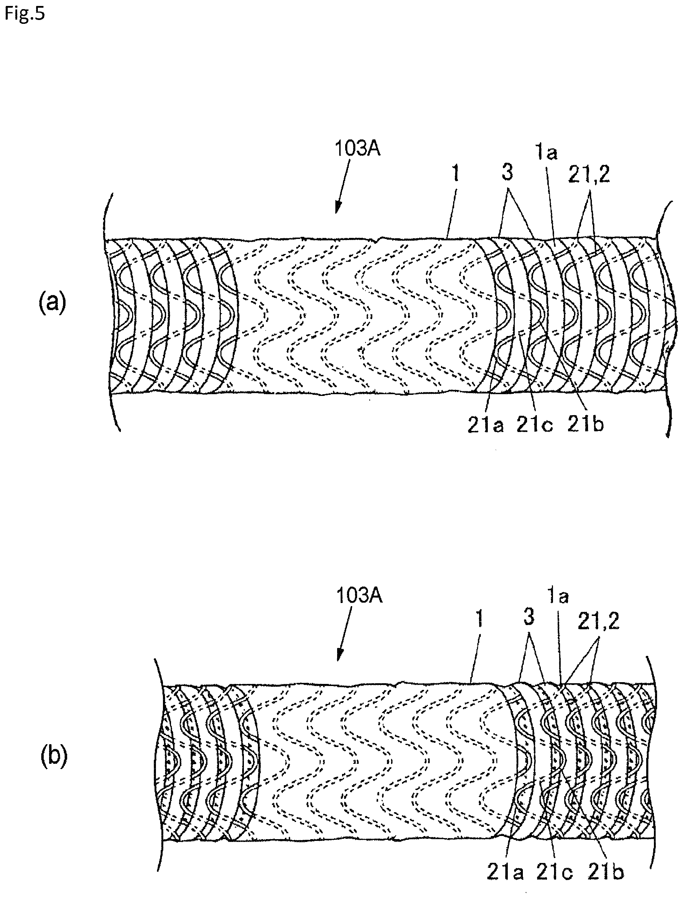

In addition, in the above embodiment, the tubular coating film part 1 is provided at both of end parts of the tubular implanted appliance 103, and the plurality of cover parts 3 are provided at the center of the tubular implanted appliance 103 (see FIG. 2(a) and FIG. 2(b)). However, the arrangement of the coating film part 1 and the cover parts 3 is an example and is not limited to this, and it is sufficient for the tubular coating film part 1 to be provided on at least part of the axial direction of the tubular implanted appliance 103, and the plurality of cover parts 3 to cover the remaining parts. For example, as shown in FIG. 5(a), the tubular coating film part 1 may be provided on the center of the tubular implanted appliance 103A, and the plurality of cover parts 3 may be provided covering both of end parts so as to sandwich the coating film part 1 in the axial direction. FIG. 5(b) schematically shows a state where the tubular implanted appliance 103A is contracted in the axial direction in a similar manner to FIG. 2(b). Moreover, the arrangement of the tubular coating film part 1 and the plurality of cover parts 3 is determined so that, for example, the coating film part 1 exists at the position of the treatment site such as an aortic aneurysm when the tubular implanted appliance 103 or 103A is implanted.

In addition, the embodiment disclosed here is to be considered in all respects as illustrative and not restrictive. The scope of the present invention is defined by the scope of the claims rather than the description above, and is intended to include any modifications within a meaning and scope equivalent to the scope of the claims.

Priority is claimed on Japanese Patent Application No. 2017-253190, filed December 28, 2017, the content of which is incorporated herein by reference.

According to the tubular implanted appliance of the present invention, treatment targets of a variety of different sizes can be handled on a per-patient basis. The present invention having this effect can be used, for example, for treating an aortic aneurysm.

### DESCRIPTION OF REFERENCE NUMERALS

- 100: Device for implanting a tubular implanted appliance
- 103: Tubular implanted appliance
- 1: Coating film part
- 1a: Exposed portion
- 2: Framework part
- 21: Linear member
- 21a, 21b: Bent part
- 21c: Intermediate portion
- 3: Cover part

## Claims

1. A tubular implanted appliance comprising a framework part and a coating film part in a tubular shape forming a partition wall, and being capable of expanding and contracting in an axial direction of the coating film part, wherein
the framework part includes a plurality of linear members in an annular shape extending along a circumferential direction of the coating film part while bending in the axial direction of the coating film part, and
the plurality of linear members are disposed in a row in the axial direction of the coating film part, and are attached and fixed to a surface of the coating film part at intermediate portions between both of end parts in the axial direction of each of the plurality of linear members.

2. The tubular implanted appliance according to claim 1, wherein
when the tubular implanted appliance is in a state where the tubular implanted appliance is contracted in the axial direction, a section of the coating film part in which the plurality of linear members are not attached or fixed becomes folded inside in a radial direction thereof.

3. The tubular implanted appliance according to claim 1 or 2, further comprising a plurality of cover parts that cover the intermediate portions of each of the plurality of linear members, wherein
the intermediate portions of each of the plurality of linear members are attached and fixed to the surface of the coating film part by attaching and fixing each of the plurality of cover parts to the surface of the coating film part.

4. The tubular implanted appliance according to claim 3, wherein
when the tubular implanted appliance is in a state where the tubular implanted appliance is contracted in the axial direction, the end part in the axial direction of one linear member among the plurality of linear members overlaps with the cover part corresponding to another linear member, which is adjacent to the one linear member, and become disposed on the surface of the cover part.

5. A tubular implanted appliance comprising a framework part and a coating film part in a tubular shape forming a partition wall, and being capable of expanding and contracting in an axial direction of the coating film part, wherein
the framework part is formed by weaving linear members in a manner that bent parts thereof, which bend in the axial direction of the coating film part, mutually engage, and is attached and fixed to a surface of the coating film part via sections other than the bent parts of the linear members.

6. A device for implanting a tubular implanted appliance comprising
a tubular implanted appliance according to any one of claims 1 to 5, wherein
the tubular implanted appliance is configured to be capable of expansion in a radial direction.
